# EUROPÄISCHE PATENTSCHRIFT

(11) **EP 1 380 316 B1**
(45) Veröffentlichungstag und Bekanntmachung des Hinweises auf die Patenterteilung: **17.10.2007**
(21) Anmeldenummer: 03400039.8
(22) Anmeldetag: 09.07.2003
(51) Int. Cl.: A61M 5/32, A61B 19/02, B65F 1/16

(54) **Sammel- und Entsorgungsbehälter, insbesondere für Kanülen**
Collect and waste container, especially for cannulas
Conteneur de collection et de déchet, spécialement pour des canules

(30) Priorität: 11.07.2002 DE 10231564
(43) Veröffentlichungstag der Anmeldung: 14.01.2004
(73) Patentinhaber: Rigling, Heinz, D-75382 Althengstett (DE)
(72) Erfinder: Rigling, Joachim, 75382 Althengstett (DE)
(74) Vertreter: Behrmann, Niels

(56) Entgegenhaltungen:
- EP-A- 0 367 422
- EP-A- 0 621 048
- EP-A- 0 691 279
- EP-A- 0 860 174
- EP-A- 0 903 158
- US-A- 5 507 408
- US-A- 6 024 217

## Beschreibung

Die Erfindung betrifft einen Sammel- und Entsorgungsbehälter mit den im Oberbegriff des Patentanspruchs 1 angegebenen Merkmalen.

Solche, auch Kanülenbehälter genannte, mit einem nach dem üblicherweise erfolgten Aufrasten nicht wieder entfernbaren Deckel verschlossene Behälter, wobei der Deckel eine verschließbare Einwurföffnung aufweist, werden z. B. in Spielzeugeimergröße in Arztpraxen und Krankenhäusern stationär und ambulant verwendet, um medizinischen Kleinabfall, wie z. B. Skalpelle, Blutpipetten, Tupfer usw., bis zur Entsorgung zu sammeln.

Um damit auch Kanülen von Injektionsspritzen sammeln zu können, die entweder unten in den Spritzenkörper eingesteckt oder von daran angeschraubten Kanülenhaltern gehalten sind, sind z. B. in einer in der Einwurföffnung ausgebildeten Art Materialvorsprung ggf. mehrere, unterschiedlich ausgestaltete Kanülen-Abzugs- bzw. Kanülen-Abtrennausnehmungen ausgebildet.

Bis zum endgültigen, gegen ein erneutes Öffnen gesicherten Verschluss der Einwurföffnung kann diese immer wieder zeitweilig, z. B. vor Behälter-Benutzungspausen, so verschlossen werden, dass sie leicht wieder zu öffnen ist. Statt aber die Einwurföffnung selbst so zu verschließen, kann z. B. auch nur der Zugang zu ihr durch ein zeitweiliges und dann endgültiges Abdecken mit einer Art Hilfsdeckel versperrt werden.

So ist es z. B. aus der Druckschrift DE 295 14 796 U1 bekannt, die kreisrunde Einwurföffnung von einer Art Halterahmen zu umgeben, der stutzenartig wie ein Kragen von der Oberseite des Behälterdeckels hochstehend an diesem angeformt ist. Er nimmt ein deckelartiges Verschlusselement so auf, das statt der Einwurföffnung die von ihm gebildete Öffnung zeitweilig oder endgültig verschlossen werden kann. Dieses ist dafür napfartig vertieft ausgebildet und wird, durch entsprechend zusammenwirkende Rastausgestaltungen an Halterahmen und Verschlusselement vorgegeben, unterschiedlich tief in den Halterahmen eingedrückt, wofür beide Hände benötigt werden.

Bei offener, d. h. nicht abgedeckter Einwurföffnung steht das üblicherweise mittels eines federnden Kunststoff-Haltebandes unverlierbar am Behälterdeckel gehaltene Verschlusselement seitwärts vom Behälterdeckel ab. Seine Unterseite, die sich während des zeitweiligen Verschlusses der Halterahmen-Öffnung in der Nähe der Kanülenabtrennausgestaltung befindet, weist dann nach oben zum Benutzer des Behälters hin. Außer eines u. U. unästhetischen Anblicks ist dies in hygienischer Hinsicht nicht unbedenklich, denn es besteht die Gefahr, diese Unterseite zu berühren, aber auch daran hängen zu bleiben und dadurch ggf. den Behälter, wenn er nicht auf einer Unterlage befestigt ist, umzureißen.

Ein Behälter nach dem Oberbegriff des Hauptanspruchs ist aus der EP 0 860 174 A1 bekannt.

Der Erfindung liegt das Problem zugrunde, einen Behälter der gattungsgemäßen Art so auszugestalten, dass eine kleine, wenig voluminöse, speziellen Benutzungsanforderungen unter ungünstigen Bedingungen, wie z. B. bei Rettungsdiensten, Notund Unfallärzten, genügende Bauform möglich ist.

Dieses Problem wird mit den im Patentanspruch 1 angegebenen Merkmalen gelöst. Vorteilhafte Weiterbildungen sind Gegenstand der Unteransprüche.

Dadurch, dass der Halterahmen und das Verschlusselement eine bewegliche Abdeck- und Verschlusseinheit für die Einwurföffnung bilden, wofür beide als lose Teile ausgebildet sind und das Verschlusselement in den Halterahmen eingesetzt und in ihm aus der zeitweiligen oberen in die endgültige untere Anordnungsposition verschiebbar gehalten ist, und dass die Abdeckund Verschlusseinheit zwischen einer die Einwurföffnung abdeckenden Stellung und einer die Einwurföffnung wenigstens überwiegend freigebenden Stellung verschiebbar oder drehbar auf dem Behälterdeckel angeordnet ist, für welche Bewegungen sich das Verschlusselement in seiner oberen Anordnungsposition im Halterahmen befindet, wird Folgendes erreicht:
- eine bei Bedarf sehr platzsparende kompakte Bauform für die hier betrachteten Sammel- und Entsorgungsbehälter, wenn die Öffnungsweite der Einwurföffnung in einer diesbezüglich optimalen geometrischen Form an die geringe Breite bestimmter, in den Behälter einzuwerfenden Gegenständen, wie z. B. bei den diesbezüglich nur eine kleine lichte Weite erfordernden, gewissermaßen schlanken Spritzenkanülen, angepasst wird, so dass dann z. B. die Länge des Verschiebeweges für das Verschieben der erfindungsgemäß an Stelle eines, wie im Stand der Technik, starren, auf der Oberseite des Behälterdeckels angeformten Halterahmens nun beweglichen Abdeck- und Verschlusseinheit höchstens dem größten Durchmesser der Einwurföffnung zu entsprechen braucht, um die Einwurföffnung für das Einwerfen der Gegenstände freizugeben;
   wird eine so gestaltete Einwurföffnung außerdem auch noch außermittig in einer Randlage auf dem Behälterdeckel positioniert und zudem die Abdeck- und Verschlusseinheit drehbar darauf angeordnet, so kann die schon für das Verschieben geringe Wegelänge noch weiter verkürzt werden, wodurch dann für bestimmte Behälter, wie z. B. bei den nur für einen einzelnen Einsatz von Rettungsdiensten, Notärzten oder Unfallärzten vorgesehenen Kanülenbehältern, sehr kleine Baugrößen mit zudem auch noch flacher statt der üblichen platzbeanspruchenden, im wesentlichen zylindrischen Form realisiert werden kann; diese können dadurch problemlos auch in einem kleinen Notfall- oder Unfall-Arztkoffer untergebracht werden und rollen z. B. nach einem Umstoßen im meist hektisch verlaufenden Notfall-Einsatz, z. B. an einer Straßensteigung, nicht weg, wie es dagegen bei zylindrischen Behältern der Fall wäre;
- vor Benutzungspausen des Behälters kann der Zugang zur Einwurföffnung durch ein leicht auszuführendes Verschieben oder Drehen der Abdeck- und Verschlusseinheit auf dem Behälterdeckel in ihre die Einwurföffnung überdeckende Stellung auf einfache bequeme Weise zeitweilig versperrt werden, ohne hierfür, wie im erwähnten Stand der Technik, eine Art Hilfsdeckel in eine vorläufige Verschlussposition in einem Halterahmen bringen zu müssen, was die Handhabung eines erfindungsgemäß ausgestatteten Behälters vereinfacht und bequemer macht;
   durch eine genauso einfache, entgegengesetzt gerichtete Bewegung der Abdeck- und Verschlusseinheit kann die Einwurföffnung wieder zum Behälter-Einfüllen zugänglich gemacht werden, und zwar beliebig oft, ohne etwa hierdurch einen Verschlussmechanismus abzunutzen wie auch ohne die von einem, wie im oben erwähnten Stand der Technik, seitwärts hoch- bzw. abstehenden Art Hilfsdeckel ausgehenden ästhetische und hygienische Beeinträchtigungen bzw. Gefahren; denn bei diesem erfindungsgemäßen Verschieben der Abdeck- und Verschlusseinheit auf dem Behälterdeckel behält diese ihre gegenüber der Behälterdeckel-Oberseite üblicherweise parallele Ausrichtung bei, d. h. ihre Unterseite, insbesondere die des Verschlusselements, bleibt in jeder ihrer durch das Verschieben einnehmbaren Stellung nach unten zur Oberseite des Behälterdeckels gerichtet und ist so, je nach Größe des Behälterdeckels, im wesentlichen unzugänglich wie auch, in üblicher vertikaler Benutzungsstellung des Behälters, unsichtbar für den Behälter-Benutzer;
- ist es im erwähnten Stand der Technik, wenn der Behälter nicht auf einer Unterlage befestigt ist, zum Zugänglichmachen der Einwurföffnung normalerweise notwendig, ihn mit einer Hand festzuhalten und mit der anderen Hand das Verschlusselement aus seiner zeitweiligen Verschlussposition im Halterahmen herauszuziehen, so kann jetzt, zumindest bei einer dies ermöglichenden kleineren Behältergröße und insbesondere drehbar angeordneter Abdeck- und Verschlusseinheit, das Öffnen bzw. Zugänglichmachen der Einwurföffnung sozusagen in Einhandbedienung, z. B. mit dem Daumen einer Hand, erfolgen, indem allein mit dem Daumen die Abdeck- und Verschlusseinheit von der Einwurföffnung weggedreht und auch wieder bis über sie zurückgedreht werden kann;
- umgekehrt muss auch zum zeitweiligen Abdecken der Einwurföffnung die Abdeck- und Verschlusseinheit nun lediglich in ihre diesbezügliche Stellung geschoben bzw. gedreht, nicht aber etwa wie ein Hilfsdeckel in einen Halterahmen eingedrückt werden, bei dem hierbei immer auch die Gefahr besteht, dass er wegen des dafür erforderlichen Kraftaufwandes, über die im Halterahmen zeitweilige, durch bestimmungsgemäß mehr oder weniger leicht überwindbare Rastausgestaltungen vorgegebene obere Verschlussposition hinaus in seine endgültige, unterhalb der zeitweiligen Verschlussposition in diesem ausgebildeten untere Verschlussposition gedrückt wird, wodurch dann der Zugang zur Einwurföffnung endgültig versperrt und der Behälter nicht weiter benutzbar ist;
   andererseits ist zum Eindrücken eines solchen Hilfsdeckels in seine endgültig die Halterahmen-Öffnung durch Verrasten dicht verschließende untere Position ein Kraftaufwand erforderlich, so dass der Behälter mit einer Hand gehalten werden muss, um dessen hierbei Umfallen zu vermeiden;
   die nun erfindungsgemäß zwischen zwei Stellungen verschiebbare bzw. drehbare Abdeck- und Verschlusseinheit vereinfacht und erleichtert somit die Handhabung wie auch die Benutzungsästethik solcher Behälter.

Dadurch, dass auf der Unterseite des Behälterdeckels eine stutzenförmig nach unten abstehende, die Kontur der Einwurföffnung umrahmende Art Verschlussrahmen ausgebildet ist und dass das Verschlusselement in seiner in der die Einwurföffnung abdeckenden Stellung der Abdeck- und Verschlusseinheit einnehmbaren unteren Anordnungsposition mit einem an die Kontur der Einwurföffnung angepassten unteren Endabschnitt durch die Einwurföffnung hindurch in den Verschlussrahmen eingreift, wobei an diesem und am Verschlusselement zumindest zur zusätzlichen Arretierung des Verschlusselements vorgesehene Arretierungsausgestaltungen wirksam werden, wird erreicht, dass nach dem für Benutzungspausen lediglich zeitweiligen Abdecken der Einwurföffnung, in der sie ja nicht wirklich, insbesondere nicht dicht verschlossen ist, nun für die Entsorgung des Behälters richtig - endgültig - verschlossen ist, d. h. nicht wieder geöffnet werden kann. Für diesen echten Verschluss der Einwurföffnung selbst braucht lediglich das Verschlusselement mit einem gewissen Kraftaufwand aus seiner oberen in seine untere Anordnungsposition im Halterahmen gedrückt zu werden.

In dieser unteren, von außen durch den Halterahmen geschützten Anordnungsposition des Verschlusselements ist nicht nur die Abdeck- und Verschlusseinheit selbst durch dessen Eingriff in den Verschlussrahmen gegen jedwede Verschiebe- bzw. Drehbewegung blockiert, sondern kann das Verschlusselement in dieser Anordnungsposition aufgrund sowohl seines Gehaltenseins im Halterahmen als auch durch die im Verschlussrahmen haltend wirksam werdende Arretierungsausgestaltung zusätzlich gesicherten unteren Lage mangels entsprechender Angriffsflächen auch nicht etwa wieder nach oben herausgezogen werden.

Darüber hinaus kann in der unteren Anordnungsposition des Verschlusselements im Halterahmen, bei entsprechender Ausbildung der Arretierungsausgestaltungen im Verschlussrahmenbereich, die Einwurföffnung gleichzeitig auch abdichtend, ggf. sogar flüssigkeitsdicht von ihm verschlossen sein.

Um auf einfache Weise eine Führung für das Verschlusselement für seine Überführung aus seiner oberen in seine untere Anordnungsposition zu erreichen, das z. B. in einem oberen Abschnitt eine andere Form als in dem durch die Einwurföffnung in den Verschlussrahmen eingreifenden unteren Abschnitt aufweisen kann, woran die Kontur des Halterahmens anpassbar wäre, ist mit Vorteil der es aufnehmende Halterahmen von einer - wie bei einem liegenden Fassreifen - hoch stehenden Wandung gebildet.

Vorzugsweise ist dieser Halterahmen an seinem oberen Rand flanschartig nach innen gerichtet verbreitert, wodurch das Verschlusselement in seiner oberen Lage in seinem hierfür ebenen Randbereich entsprechend überdeckt und gegen ein evtl. Herausfallen aus dem Halterahmen gesichert ist. Dadurch bleibt auch die zweiteilige Ausgestaltung der Abdeck- und Verschlusseinheit aus Halterahmen und darin verschiebbar angeordnetem Verschlusselement zunächst verborgen.

Mit Vorteil ist die Einwurföffnung, angepasst an die Größenordnung der in einen Behälter einzuwerfenden Gegenstände, in Form einer dafür platzsparend ausbildbaren Ellipse ausgebildet und die daran angepasst elliptische Abdeck- und Verschlusseinheit auf dem dann vorteilhaft ebenfalls elliptischen Behälterdeckel drehbar angeordnet. Hierdurch kann durch die Drehbeweglichkeit der Abdeck- und Verschlusseinheit eine wenig Platz beanspruchende Abdeck- und Verschlussvorrichtung für die Einwurföffnung geschaffen werden, die während der Behälterbenutzung zwischen einer die Einwurföffnung freigebenden und einer sie zeitweilig abdeckenden Stellung auf kleinstem Raum hin und her gedreht werden kann. Dadurch wird eine besonders kleine kompakte flache, zudem ein Wegrollen umgekippter oder umgestoßener Behälter verhindernde Bauform für z. B. einen Kanülenbehälter möglich. Sie lässt auch eine in bestimmten Situationen erwünschte Einhandbedienung in Bezug auf das zeitweilige Öffnen und Abdecken der Einwurföffnung, z. B. mit dem Daumen, zu, wie auch beim endgültigen Verschließen der Einwurföffnung durch Eindrücken des Verschlusselements in seine untere Anordnungsposition im Halterahmen.

Anhand von schematischen Zeichnungen wird nachfolgend als bevorzugtes Ausführungsbeispiel der Erfindung ein Kanülen-Sammel- und Entsorgungsbehälter beschrieben, wobei sich weitere Einzelheiten und Vorteile der Erfindung ergeben.

Es zeigen:
- Fig. 1: eine geringfügig vergrößerte perspektivische Ansicht des Kanülenbehälters mit offener Einwurföffnung,
- Fig. 2: eine perspektivische Ansicht des Behälters nach Fig. 1 mit von der Abdeck- und Verschlusseinheit abgedeckter Einwurföffnung,
- Fig. 3: eine weiter vergrößerte Draufsicht auf den Behälter nach Fig. 1,
- Fig. 4: eine ebenfalls weiter vergrößerte Draufsicht auf den Behälter nach Fig. 2,
- Fig. 5: eine teilweise aufgebrochene und auch geschnittene Teil-Seitenansicht des Behälters nach Fig. 2 und
- Fig. 6: eine teilweise aufgebrochene und auch geschnittene Teil-Seitenansicht des Behälters nach Fig. 5 mit dem Verschlusselement der Abdeck- und Verschlusseinheit in seiner die Einwurföffnung endgültig verschließenden unteren Anordnungsposition.

Der in den Figuren in Vergrößerung gezeigte kleine, leicht konisch nach oben sich erweiternde, aufgrund seines elliptischen Querschnitts flache Behälter hat eine Gesamthöhe von lediglich ca. 132 mm und oben eine Gesamtbreite von ca. 74 mm und Gesamttiefe von nur ca. 48 mm.

Durch diese spezielle Ausgestaltung eignet sich der gezeigte Behälter besonders gut zur Bestückung der eine nur beschränkte Aufnahmefähigkeit solcher Utensilien aufweisenden Ärztetaschen oder Ärztekoffer, vorrangig derjenigen von Notärzten, Unfallärzten wie auch sonstigen Rettungsdiensten. Da diese solche Behälter nur jeweils während eines Einsatzes benutzen, u. a. auch zum Sammeln und Entsorgen von so genannten Venenverweilkanülen, ist ein Behälter dieser Größenordnung in diesem Einsatzbereich auch aus Kostengründen erwünscht.

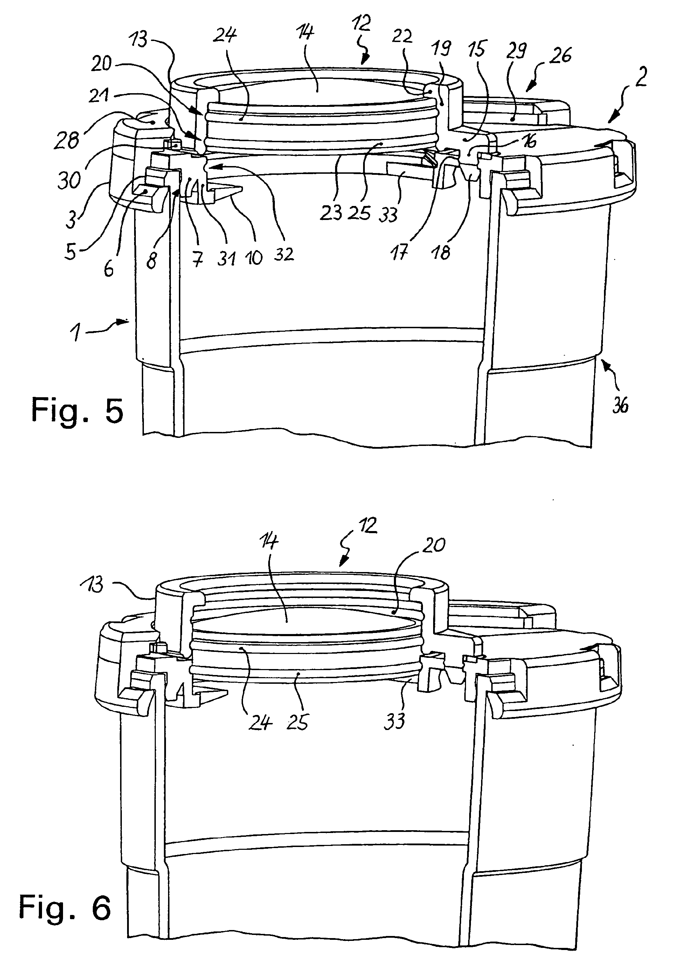

Der zur Entsorgung durch Verbrennen aus einem entsprechenden Kunststoff bestehende Behälter hat ein Behälterunterteil 1 und einen unlösbar darauf aufgerasteten, im wesentlichen ebenen Behälterdeckel 2 mit hierfür einem am Umfang senkrecht nach unten abstehend ausgebildeten Deckelrandkragen 3. Die darin in fensterartigen Ausnehmungen 4 nach innen vorstehend ausgebildeten Rastnocken 5 sind unter einen am Öffnungsrand des Behälterunterteils ausgebildeten Randflansch 6 eingerastet (Fig. 5).

Ein weiterer, auf der Unterseite des Behälterdeckels 2 in einem Abstand vom Deckelrandkragen 3 stutzenartig senkrecht nach unten abstehend umlaufender Kragen 7 mit einer auf seiner Außenseite ausgebildeten, mit der Innenwandung des Behälterunterteils zusammenwirkenden Dichtungswulst 8 (Fig. 5) bewirkt in Verbindung mit dem Deckelrandkragen 3 die Dichtigkeit dieses Behälterdeckel-Verschlusses.

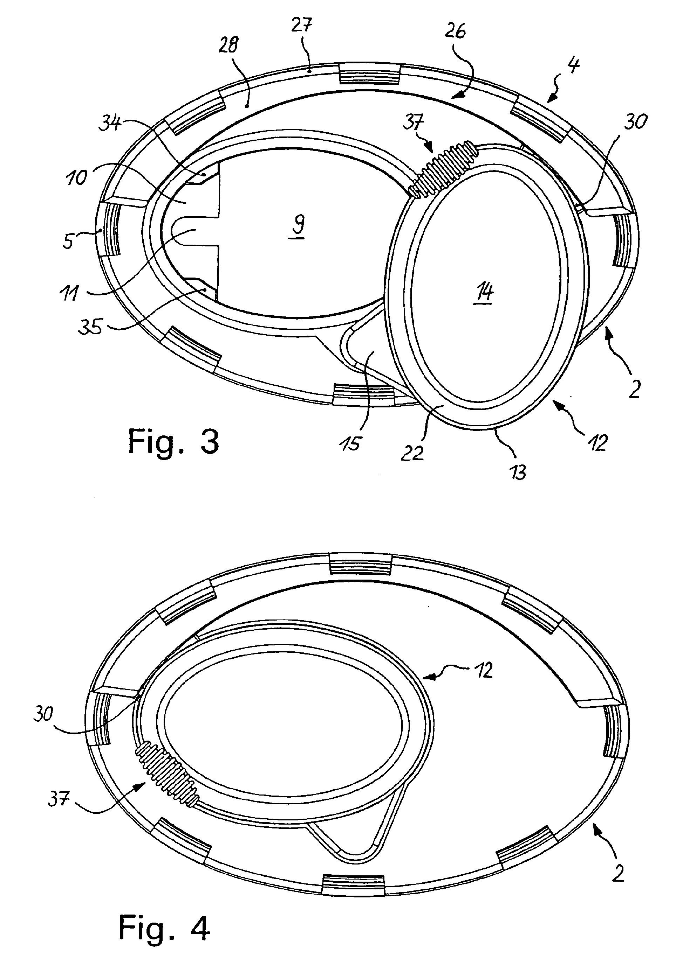

Die Behälter-Einwurföffnung 9 ist, in Anpassung an die Abmessungen der einzuwerfenden Gegenstände, das sind vorrangig im wesentlichen schlanke Spritzenkanülen, in Form einer Ellipse links (Fig. 3) in einer Randlage des dementsprechend ebenfalls elliptisch ausgebildeten Behälterdeckels 2 so angeordnet, dass deren Ellipsen-Hauptachsen zusammenfallen und sich ihre linken Ellipsen-Hauptscheitel in unmittelbarer Nachbarschaft befinden.

Durch eine in einem links (Fig. 3) in die Einwurföffnung 9 vorstehenden Materialvorsprung 10 ausgebildete Kanülenabzugsausnehmung 11 können in das untere Ende von Injektionsspritzen eingesteckte Kanülen nach der Benutzung gefahrlos abgezogen werden, damit sie von selbst in den Behälter fallen. Anstelle dieser Abzugsausnehmung 11 könnte in diesem Materialvorsprung 10 auch eine lochförmig geschlossene Abtrennausnehmung mit z. B. von ihrem Innenrand fingerartig abstehenden Festklemmelementen zum Abdrehen von unten am Spritzenkörper angeschraubten Kanülenhalter ausgebildet sein.

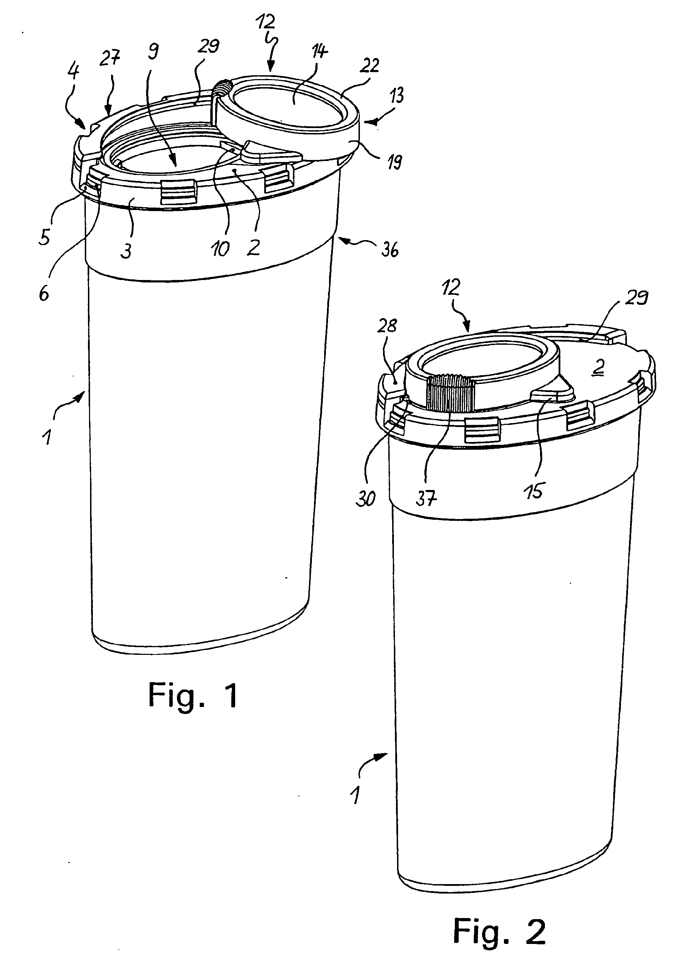

Zum zeitweiligen Abdecken der Einwurföffnung 9, z. B. für Behälter-Benutzungspausen, sowie zum endgültigen Verschluss der Einwurföffnung 9 für die Behälter-Entsorgung, ist auf dem Behälterdeckel 2 eine in Anpassung an die Einwurföffnung 9 ebenfalls elliptisch ausgebildete Abdeck- und Verschlusseinheit 12 vorgesehen. Sie ist von einem losen Halterahmen 13 und einem losen kappenförmigen Verschlusselement 14 gebildet, das von unten in den Halterahmen 13 eingesetzt und in ihm positionsveränderlich zwischen einer zeitweiligen oberen (Fig. 1 bis 5) und einer daraus endgültig einnehmbaren unteren Anordnungsposition (Fig. 6) gehalten ist.

Die Abdeck- und Verschlusseinheit 12 kann zwischen einer Stellung, in der sie die Einwurföffnung 9 wenigstens annähernd vorrangig zum Kanülenabziehen oder auch Kanülenabtrennen, aber auch zum Einwerfen sonstiger, entsprechend kleiner Gegenstände in den Behälter freigibt (Fig. 1 und 3), und einer Stellung, in der sie die Einwurföffnung 9 völlig abdeckt und so den Zugang zu dieser versperrt (Fig. 2 und 4), auf dem Behälterdeckel 2 hin und her gedreht werden.

Hierfür ist rechts (Fig. 4) am unteren Rand des Halterahmens 13 eine Lasche 15 senkrecht von ihm abstehend angeformt, von deren Unterseite ein Drehzapfen 16 senkrecht nach unten absteht. Er ist in eine in der Nähe des einen Nebenscheitels des elliptischen Behälterdeckels 2 ausgebildete Ausnehmung in den Behälterdeckel 2 eingedrückt.

In dem Abschnitt seines Schaftes, mit dem er durch den Behälterdeckel 2 hindurchtritt (Fig. 5), ist an seiner Wandung eine Dichtungswulst 17 ausgebildet, die mit der Wandung der Ausnehmung, letztere gegenüber dem Behälterinneren abdichtend, zusammenwirkt.

Mit der an der Unterseite des Behälterdeckels 2 anliegenden Oberseite seines verdickten, konisch sich verjüngenden, für die Montage axial geschlitzten freien Schaftendes 18 ist der Drehzapfen 16 im Behälterdeckel 2 arretiert.

Der Halterahmen 13 wird von einer vertikal fassreifenartig hoch stehenden Wandung 19 gebildet, auf deren Innenseite, voneinander beabstandet, eine horizontal umlaufende obere und untere Rastnut 20, 21 ausgebildet sind. Vom oberen Rand der Wandung 19 steht eine flanschartig nach innen gerichtete Verbreiterung 22 ab, um eine den ebenen Randbereich der ansonsten konvex ausbildeten Abdeckfläche des Verschlusselements 14 überdeckende Anlage zu bilden.

Auf der Außenseite der gegenüber der Wandung 19 des Halterahmens 13 kürzeren vertikalen Seitenwandung 23 des Verschlusselements 14 sind zum Zusammenwirken mit den beiden Rastnuten 20, 21 des Halterahmens 13 eine entsprechende obere und untere Rastrippe 24, 25, gleichermaßen beabstandet, komplementär ausgebildet. Sie sind in der in den Fig. 1 bis 5 gezeigten oberen Anordnungsposition des Verschlusselements 14 im Halterahmen 13 in dessen beide Rastnuten 20, 21 eingerastet und halten so das in dieser Lage völlig in den Halterahmen 13 aufgenommene Verschlusselement 14, so dass die Abdeck- und Verschlusseinheit 12 behinderungsfrei zwischen ihrer die Einwurföffnung 9 freigebenden (Fig. 1 und 3) und sie abdeckenden Stellung (Fig. 2, 4 und 5) auf dem Behälterdeckel 2 hin und her gedreht werden kann.

Auf dem der den Drehzapfen 16 im Behälterdeckel 2 aufnehmenden Ausnehmung gegenüberliegenden Rand des Behälterdeckels 2 ist annähernd am halben Deckelumfang eine Führung 26 für die Abdeck- und Verschlusseinheit 12 ausgebildet. Sie wird von einer randseitig vom Behälterdeckel 2 hoch stehenden, den nach unten abstehenden Randkragen 3 des Behälterdeckels 2 in etwa nach oben verlängernd ausgebildeten Art Materialrippe 27 mit einem auf der Innenseite vertikalen, kreisbogenförmig nach innen gekrümmten Wandungsverlauf und einer an ihrem oberen Ende waagerechten, einwärts weisenden rippenförmigen Verbreiterung 28 gebildet. Letztere bildet mit der Oberseite des Behälterdeckels 2 eine Führungsnut 29, wobei der Mittelpunkt des Kreisbogens und die Drehachse des Drehzapfens 16 der Abdeck- und Verschlusseinheit 12 zusammenfallen.

In diese Führung 26 greift ein unten am Rand des Halterahmens 13 in der Nähe seines linken Ellipsen-Hauptscheitels der Abdeck- und Verschlusseinheit 12 nasenartig abstehend ausgebildeter Materialvorsprung 30 zur Führung der Abdeck- und Verschlusseinheit 12 bei ihren Drehbewegungen ein.

Diese Führung 26 entlastet den Drehzapfen 16 beim Drehen der Abdeck- und Verschlusseinheit 12 und erhöht die Sicherheit des endgültigen Verschlusses der Einwurföffnung 9 nach dem Eindrücken des Verschlusselements 14 in seine untere Anordnungsposition im Halterahmen 13, indem der dann im wesentlichen unzugänglich in die Führungsnut 29 aufgenommene Materialvorsprung 30 des Halterahmens 13 z. B. ein Hochdrücken der Abdeck- und Verschlusseinheit 12 auf der dem Drehzapfen 16 schräg gegenüberliegenden Seite des Halterahmens 13 verhindert kann.

In der die Einwurföffnung 9 freigebenden Stellung der Abdeckund Verschlusseinheit 12 (Fig. 1 und 3) kann ein - nicht gezeigter - Anschlag, z. B. am Ende der Führungsnut 29, verhindern, dass die Abdeck- und Verschlusseinheit 12 weiter als in Fig. 3 gezeigt über den Behälterdeckel 2 hinaus gedreht wird.

In der die Einwurföffnung 9 abdeckenden Stellung der Abdeckund Verschlusseinheit 12 (Fig. 1, 3, 5 und 6) kann z. B. ein von der Oberseite des Behälterdeckels 2 hoch stehend ausgebildeter - nicht gezeigter - Nocken in eine entsprechende Ausnehmung des in dieser Stellung dann mit einem kurzen Endabschnitt links (Fig. 4) aus der Führungsnut 29 herausgetretenen Materialvorsprungs 30 des Halterahmens 13 einrasten und, da zur Aufhebung einer solchen Verrastung ein gewisser Kraftaufwand notwendig ist, die Abdeck- und Verschlusseinheit 12 in dieser Abdeck-Stellung halten und somit verhindern, dass sie eine ungewollte, zu einem Freigeben der Einwurföffnung 9 führende Drehbewegung ausführt.

Auf der Unterseite des Behälterdeckels 2 ist die Einwurföffnung 9 von einer stutzenförmig senkrecht nach unten abstehend die Kontur der Einwurföffnung 9 einrahmend ausgebildeten Art Verschlussrahmen 31 eingefasst. Auf dessen Innenseite, in einem von den beiden Rastnuten 20, 21 des Halterahmens 13 vorgegebenen Abstand von der unteren Rastnut 21 des Halterahmens 13 entfernt, ist eine dritte Rastnut 32 komplementär zu der unteren Rastrippe 25 des Verschlusselements 12 ausgebildet.

In der in Fig. 6 gezeigten unteren, aus der oberen durch einen Kraftaufwand zur Überwindung der doppelten Verrastung 20, 24 und 21, 25 einnehmbaren Anordnungsposition des Verschlusselements 14, sind dessen obere Rastrippe 24 in die untere Rastnut 21 des Halterahmens 13 und untere Rastrippe 25 in die Rastnut 32 des Verschlussrahmens 31 eingerastet und so das Verschlusselement 14 zum endgültigen Verschluss der von der Abdeck- und Verschlusseinheit 12 abgedeckten Einwurföffnung 9 zweifach irreversibel gehalten und arretiert.

Durch den so herbeigeführten Eingriff des Verschlusselements 12 in den Verschlussrahmen 31 ist nicht nur die Abdeck- und Verschlusseinheit 12 gegen jede Drehbewegung gesichert, sondern durch diese - formschlüssige - Verrastung von unterer Rastrippe 25 des Halterahmens 13 mit der Rastnut 32 des Verschlussrahmens 31 gleichzeitig auch die Einwurföffnung 9 nach außen hin abgedichtet.

Unterhalb der Rastnut 32, jeweils symmetrisch zur Ellipsen-Hauptachse des Verschlussrahmens 31, ist in seinem rechten Hauptscheitel-Krümmungsbereich (Fig. 5) ein geringfügig vorstehender Materialvorsprung 33 und im gegenüberliegenden Hauptscheitel-Krümmungsbereich sind jeweils in einem Abstand von der Ellipsen-Hauptachse zwei weitere, ebenfalls nur geringfügig vorstehende Materialvorsprünge 34, 35 ausgebildet. Sie bilden jeweils eine Aufsitzfläche für den unteren Rand des Verschlusselements 14, um zu verhindern, dass dieses beim Überführen aus seiner oberen in seine untere Anordnungsposition im Halterahmen 13 aufgrund des hierzu aufzubringenden Kraftaufwands über die untere Anordnungsposition hinaus noch weiter ins Behälterunterteil 1 gedrückt wird.

Unterhalb dieser drei Materialvorsprünge 33, 34 und 35 ist am unteren linken Rand des Verschlussrahmens 31 der bereits oben erwähnte größere Materialvorsprung 10 mit der Kanülenabzugsausgestaltung 11 ausgebildet.

Zur Erhöhung der Benutzungssicherheit des gezeigten Behälters ist dessen Seitenwandung unten in einem ca. ein Viertel der gesamten Höhe des Behälters ausmachenden Abschnitt verstärkt ausgeführt, um so zu verhindern, dass etwa die Spitzen von in den Behälter eingeworfenen Kanülen durch die Wandung nach außen durchdrücken.

In einem Abstand unterhalb des oberen Randes des Behälters ist ein horizontal umlaufender Verbreiterungsabsatz 36 in der Seitenwandung des Behälterunterteils ausgebildet. Er stellt eine obere Füllstandsmarkierung für den Behälter dar, die beim Einwerfen durch die offene Einwurföffnung 9 sichtbar ist und beim Befüllen des Behälters, insbesondere beim Einwerfen von Kanülen, beachtet werden soll, damit das Verschlusselement 14 zum Verschluss der Einwurföffnung 9 zur Entsorgung des Behälters unbehindert in seine untere Anordnungsposition im Halterahmen 13 eingedrückt werden kann.

Um die zeitweilig von der Abdeck- und Verschlusseinheit 12 abgedeckte Einwurföffnung 9 (Fig. 2 und 4) zum Einwerfen von Gegenständen zugänglich zu machen, kann diese bei z. B. mit der rechten Hand umgriffenen kleinen Behälter in Einhandbedienung mit dem Daumen in ihre andere, die Einwurföffnung 9 freigebende Stellung (Fig. 1 und 3) und daraus ebenso wieder mit dem Daumen in die Ausgangsstellung (Fig. 2 und 4) zurück gedreht werden.

Zur Verbesserung einer diesbezüglichen Griffigkeit des Halterahmens 13 ist in einem dafür griffgünstig gelegenen Randabschnitt (Fig. 4), in der Nähe des linken Ellipsen-Hauptscheitels der Abdeck- und Verschlusseinheit 12, am Halterahmen 13 eine Oberflächenausgestaltung in Form einer Folge von senkrechten, sich oben auf der nach innen gerichteten Wandungsverbreiterung 22 fortsetzend ausgebildeten Rippen 37 vorgesehen.

Durch die Idee, die lichte Weite der Einwurföffnung 9 eines Behälters auf ein notwendiges Mindestmaß zu verringern, wobei eine elliptische Form einer solchen Einwurföffnung 9 ein diesbezügliches Optimum darstellen kann, und diese elliptische Einwurföffnung in einem Randbereich eines ebenfalls elliptischen Behälterdeckels 2 anzuordnen und für das zeitweilige Abdecken und endgültige Verschließen der Einwurföffnung eine entsprechend elliptische Abdeck- und Verschlusseinheit 12 drehbar zwischen einer die Einwurföffnung 9 freigebenden und sie abdeckenden Stellung auf dem Behälter- deckel 2 vorzusehen, kann also ein platzsparend auch in beengten Behältnissen unterbringbarer Behälter geschaffen werden mit einem zumindest für einen Einsatz von Notfallärzten, Unfallärzten und Rettungsdiensten ausreichendem Volumen zur Aufnahme von gebrauchten Spritzenkanülen, auch, wie erwähnt, unter widrigen Einsatzbedingungen, unter denen aber auch umgestoßene Kanülenbehälter nicht wegrollen können sollten.

In einer solchen bevorzugten elliptischen Behälter-Ausgestaltung beträgt die Länge der Ellipsen-Haupt des Behälterdeckels 1 etwa das 1,85-fache derjenigen der Abdeck- und Verschlusseinheit 12 und die der Ellipsen-Nebenachse des Behälterdeckels 2 etwa das 1,72-fache derjenigen des Abdeck- und Verschlusseinheit 12.

### Bezugszeichenliste

- 1: Behälterunterteil
- 2: Behälterdeckel
- 3: Deckelrandkragen
- 4: Ausnehmungen
- 5: Rastnocken
- 6: Randflansch
- 7: Kragen
- 8: Dichtungswulst
- 9: Einwurföffnung
- 10: Materialvorsprung
- 11: Kanülenabzugsausnehmung
- 12: Abdeck- und Verschlusseinheit
- 13: Halterahmen
- 14: Verschlusselement
- 15: Lasche
- 16: Drehzapfen
- 17: Dichtungswulst von 16
- 18: freies Schaftende von 16
- 19: Wandung von 13
- 20: Rastnut
- 21: Rastnut
- 22: Verbreiterung von 19
- 23: Seitenwandung von 14
- 24: Rastrippe
- 25: Rastrippe
- 26: Führung für 12
- 27: Materialrippe von 26
- 28: Verbreiterung von 27
- 29: Führungsnut
- 30: Materialvorsprung von 13
- 31: Verschlussrahmen
- 32: Rastnut
- 33: Materialvorsprung, Aufsitzfläche
- 34: Materialvorsprung, Aufsitzfläche
- 35: Materialvorsprung, Aufsitzfläche
- 36: Verbreiterungsabsatz
- 37: Rippen

## Patentansprüche

1. Sammel- und Entsorgungsbehälter mit einem BehälterUnterteil (1) und einem Behälterdeckel (2) mit
- einer Einwurföffnung (9),
- einer Art Halterahmen (13), der stutzenförmig vom Behälterdeckel (2) hoch steht und für eine die Einwurföffnung umrahmende Anordnung bestimmt und angepasst ist, und
- einem Verschlusselement (14), das in der die Einwurföffnung (9) umrahmenden Anordnung des Halterahmens wenigstens für ein Abdecken der Einwurföffnung durch Verschließen der von dem Halterahmen gebildeten Öffnung vorgesehen ist, wobei für ein nur zeitweiliges Abdecken der Einwurföffnung (9) eine obere Anordnungsposition und für deren endgültiges Abdecken eine durch einen Kraftaufwand aus der oberen einnehmbare untere Anordnungsposition für das Verschlusselement im Halterahmen vorgesehen ist,
wobei
- der Halterahmen (13) und das Verschlusselement (14) eine bewegliche Abdeck- und Verschlusseinheit (12) für die Einwurföffnung (9) bilden, wofür beide als lose Teile ausgebildet sind und das Verschlusselement (14) in den Halterahmen (13) eingesetzt und in ihm aus der oberen in die untere Anordnungsposition verschiebbar gehalten ist,
- die Abdeck- und Verschlusseinheit (12) zwischen einer die Einwurföffnung (9) abdeckenden Stellung und einer die Einwurföffnung (9) wenigstens überwiegend freigebenden Stellung um eine Drehachse drehbar auf dem Behälterdeckel (2) angeordnet ist, für welche Bewegungen sich das Verschlusselement (14) in seiner oberen Anordnungsposition im Halterahmen (13) befindet,
- auf der Unterseite des Behälterdeckels (2) eine stutzenförmig nach unten abstehende, die Kontur der Einwurföffnung (9) umrahmende Art Verschlussrahmen (31) ausgebildet ist und
- das Verschlusselement (14) in seiner in der die Einwurföffnung (9) abdeckenden Stellung der Abdeck- und Verschlusseinheit (12) einnehmbaren unteren Anordnungsposition mit einem an die Kontur der Einwurföffnung (9) angepassten unteren Endabschnitt durch die Einwurföffnung (9) hindurch in den Verschlussrahmen (31) eingreift, wobei an diesem (31) und am Verschlusselement (14) zumindest zur zusätzlichen Arretierung des Verschlusselements vorgesehene Arretierungsausgestaltungen (32, 25) wirksam werden,
**dadurch gekennzeichnet, dass**
- die Einwurföffnung (9), der Halterahmen (13) und das Verschlusselement (14) eine aneinander angepasste elliptische Kontur aufweisen und die Einwurföffnung in einer Randlage des elliptisch ausgebildeten Behälterdeckels (2) so angeordnet ist, dass deren Ellipsen-Hauptachsen zusammenfallen, und
- der Behälter augrund seines elliptischen Querschnitts eine flache Bauform aufweist, die eine Einhandbedienung in Bezug auf das Drehen der Abdeck- und Verschlusseinheit zwischen der die Einwurföffnung abdeckenden Stellung und der die Einwurföffnung wenigstens überwiegend freigebenden Stellung durch den Daumen bei mit der Hand umgriffenem Behälter zulässt.

2. Behälter nach Anspruch 1, **dadurch gekennzeichnet, dass** das Verschlusselement (14) kappenförmig mit einer in Bezug auf die Oberseite des Behälterdeckels (2) senkrecht nach unten weisenden Seitenwandung (23) ausgebildet ist, auf deren Außenseite im oberen und unteren Randbereich jeweils eine horizontal umlaufende obere und untere Rastausgestaltung (24, 25) vorgesehen ist.

3. Behälter nach Anspruch 2, **dadurch gekennzeichnet,**
- **dass** der Halterahmen (13) von einer in Bezug auf den Behälterdeckel (2) senkrecht fassreifenartig hoch stehenden Wandung (19) gebildet ist, an deren oberem Rand eine flanschartig nach innen gerichtete Verbreiterung (22) ausgebildet ist, und
- **dass** auch auf der Innenseite des Halterahmens (13) in zu den beiden Rastausgestaltungen (24, 25) des Verschlusselements (14) komplementärer Weise eine obere und untere Rastausgestaltung (20, 21) ausgebildet ist.

4. Behälter nach Anspruch 3, **dadurch gekennzeichnet, dass** auf der Innenseite des Verschlussrahmens (31) in einem von den beiden Rastausgestaltungen (20, 21) des Halterahmens (13) vorgegebenen Abstand von dessen unterer Rastausgestaltung (21) entfernt, eine dritte Rastausgestaltung (32) komplementär zu der unteren Rastausgestaltung (25) des Verschlusselements (14) ausgebildet ist.

5. Behälter nach Anspruch 4, **dadurch gekennzeichnet, dass** von der Innenwandung des Verschlussrahmens (31) in einem Abstand unterhalb der Rastausgestaltung (32) eine Anzahl Aufsitzflächen (33, 34, 35) für den unteren Rand des Verschlusselements (14) in Form von geringfügig abstehenden Materialvorsprüngen ausgebildet sind.

6. Behälter nach Anspruch 5, **dadurch gekennzeichnet, dass** unterhalb der Aufsitzflächen (33, 34, 35) in einem diesen gegenüber größeren Materialvorsprung (10) wenigstens eine Kanülenabtrenn-Ausgestaltung (11) vorgesehen ist.

7. Behälter nach einem der vorhergehenden Ansprüche, **dadurch gekennzeichnet, dass** die Abdeck- und Verschlusseinheit in zwei auf dem Behälterdeckel sich gegenüberliegend ausgebildeten Führungen geführt ist.

8. Behälter nach einem der vorhergehenden Ansprüche, **dadurch gekennzeichnet, dass** die Abdeck- und Verschlusseinheit (12) in ihrer die Einwurföffnung (9) überdeckenden Stellung von einer durch Kraftaufwand überwindbaren Klemmausgestaltung gehalten ist.

9. Behälter nach einem der Ansprüche 1 bis 8, **dadurch gekennzeichnet, dass** vom unteren Rand des Halterahmens (13) eine Lasche (15) absteht, von deren Unterseite ein Drehzapfen (16) senkrecht nach unten absteht, der in eine entsprechende Ausnehmung im Behälterdeckel (2) eingedrückt ist.

10. Behälter nach Anspruch 9, **dadurch gekennzeichnet,**
- **dass** auf dem Drehzapfen (16) in dem Abschnitt, mit dem er die Ausnehmung im Behälterdeckel (2) durchquert, eine mit der Wandung der Ausnehmung zusammenwirkende Dichtungswulst (17) ausgebildet ist und
- **dass** das freie Ende (18) des Drehzapfens (16) für ein Hintergreifen der Unterseite des Behälterdeckels (2) verbreitert ist und anschließend sich konisch verjüngt, wobei der freie Endabschnitt einen mittig in Achsrichtung ausgebildeten Schlitz aufweist.

11. Behälter nach einem der vorhergehenden Ansprüche, **dadurch gekennzeichnet, dass** auf dem Behälterdeckel (2) eine zumindest in Kreisbogenform ausgebildete Führung (26) für die Abdeck- und Verschlusseinheit (12) ausgebildet ist, wobei die Drehachse (16) der Abdeck- und Verschlusseinheit (12) senkrecht durch den Mittelpunkt des Kreisbogens verläuft.

12. Behälter nach einem der Ansprüche 1 bis 11, **dadurch gekennzeichnet,**
- **dass** sich die Drehachse (16) der Abdeck- und Verschlusseinheit (12) in unmittelbarer Nachbarschaft zu einem der beiden Ellipsen-Nebenscheitel des Behälterdeckels (2) befindet.

13. Behälter nach Anspruch 12, **dadurch gekennzeichnet,**
- **dass** die Führung (26) der Abdeck- und Verschlusseinheit (12) von einer annähernd am halben Umfang des der Ausnehmung für den Drehzapfen (16) gegenüberliegenden Randes des Behälterdeckels (2) davon hoch stehende Art Materialrippe (27) mit einer von ihrem oberen Rand in Richtung zur Kreisbogenmitte hin abstehenden rippenartigen Verbreiterung (28) gebildet ist, welche mit der Oberseite des Behälterdeckels (2) eine Führungsnut (29) bildet, und
- **dass** in diese Führungsnut (29) eine vom Rand des Halterahmens (13) im Bereich des benachbarten Ellipsen-Hauptscheitels ein nasenartig abstehender Materialvorsprung (30) eingreift.

## Claims

1. Collecting and waste disposal container with a bottom part (1) and a lid (2), comprising
- a feed aperture (9),
- a type of supporting frame (13) which extends upwards from the container lid (2) in the form of a connecting piece and is intended for and adapted to an arrangement framing the feed aperture, and
- a closing element (14) provided in the arrangement of the supporting frame framing the feed aperture (9) at least to cover the feed aperture by closing the opening formed by the supporting frame, an upper configuration being provided for the closing element in the supporting frame for only temporarily covering the feed aperture (9) and a lower configuration which can be adopted by the application of force from the upper position for the final covering thereof,
wherein
- the supporting frame (13) and the closing element (14) form a movable covering and closing unit (12) for the feed aperture (9), to which end they are both designed as loose parts and the closing element (14) is inserted into the supporting frame (13) and held therein in such a manner that it can be displaced from the upper to the lower configuration,
- the covering and closing unit (12) is arranged on the container lid (2) in such a manner that it can rotate about an axis of rotation between a position covering the feed aperture (9) and a position at least substantially uncovering the feed aperture (9), for which movements the closing element (14) is situated in its upper configuration in the supporting frame (13),
- a type of closing frame (31) projecting downwards in the form of a connecting piece and framing the contour of the feed aperture (9) is provided on the underside of the container lid (2), and
- the closing element (14) engages through the feed aperture (9) into the closing frame (31) in its lower configuration which can be adopted in the position of the covering and closing unit (12) covering the feed aperture (9) by means of a lower end portion adapted to the contour of the feed aperture (9), locking arrangements (32, 25) provided at least for additional locking of the closing element on the closing frame (31) and on the closing element (14) becoming active,
**characterised in that**
- the feed aperture (9), the supporting frame (13) and the closing element (14) each have elliptical contours adapted to one another and the feed aperture is arranged in an edge position of the elliptical container lid (2) in such a manner that their major axes coincide with one another, and,
- as a result of its elliptical cross section, the container has a flat design which allows for single-handed operation with respect to rotating the covering and closing unit between the position covering the feed aperture and the position at least substantially uncovering the feed aperture by means of the thumb when the container is gripped by the hand.

2. Container according to claim 1, **characterised in that** the closing element (14) is cap-shaped with a side wall (23) directed vertically downwards with respect to the top surface of the container lid (2), in the upper and lower edge regions of the outer face of which respective horizontally circumferential upper and lower snap-in arrangements (24, 25) are provided.

3. Container according to claim 2, **characterised in that**
- the supporting frame (13) is formed by a wall (19) extending vertically upwards like a hoop with respect to the container lid (2), on the upper edge of which a broadened portion (22) directed inwards like a flange is provided, and
- upper and lower snap-in arrangements (20, 21) are also provided on the inner face of the supporting frame (13) in a complementary manner to the two snap-in arrangements (24, 25) of the closing element (14).

4. Container according to claim 3, **characterised in that** a third snap-in arrangement (32) is provided in a complementary manner to the lower snap-in arrangement (25) of the closing element (14) on the inner face of the closing frame (31) at a distance from the lower snap-in arrangement (21) predetermined by the two snap-in arrangements (20, 21) of the supporting frame (13).

5. Container according to claim 4, **characterised in that** a number of bearing surfaces (33, 34, 35) for the lower edge of the closing element (14) in the form of slightly protruding material projections are provided by the inner wall of the closing frame (31) at a distance below the snap-in arrangement (32).

6. Container according to claim 5, **characterised in that** at least one needle-separating arrangement (11) is provided below the bearing surfaces (33, 34, 35) in a larger material projection (10).

7. Container according to one of the preceding claims, **characterised in that** the covering and closing unit is guided in two guides provided opposite one another on the container lid.

8. Container according to one of the preceding claims, **characterised in that** the covering and closing unit (12) is held in its position covering the feed aperture (9) by a clamping arrangement which can be overcome by the application of force.

9. Container according to one of claims 1 to 8, **characterised in that** a tab (15) from the underside of which a pivot pin (16) extends vertically downwards and is pressed into a corresponding recess in the container lid (2) projects from the lower edge of the supporting frame (13).

10. Container according to claim 9, **characterised in that**
- a sealing bead (17) cooperating with the wall of the recess is provided on the pivot pin (16) in the portion by means of which it traverses the recess in the container lid (2) and
- the free end (18) of the pivot pin (16) is broadened for engaging behind the underside of the container lid (2) and then tapers, the free end portion having a slot provided centrally in the axial direction.

11. Container according to one of the preceding claims, **characterised in that** a guide (26) designed at least in the form of a circular arc is provided on the container lid (2) for the covering and closing unit (12), the axis of rotation (16) of the covering and closing unit (12) extending vertically through the centre of the circular arc.

12. Container according to one of claims 1 to 11, **characterised in that** the axis of rotation (16) of the covering and closing unit (12) is situated in the immediate vicinity of one of the two vertices of the minor axis of the ellipse of the container lid (2).

13. Container according to claim 12, **characterised in that**
- the guide (26) of the covering and closing unit (12) is formed by a type of material rib (27) extending upwards from approximately half the circumference of an edge of the container lid (2) situated opposite the recess for the pivot pin (16) with a rib-like broadened portion (28) protruding from its upper edge in the direction of the centre of the circular arc which forms a guide groove (29) together with the top surface of the container lid (2), and
- a material projection (30) protruding like a lug from the edge of the supporting frame (13) in the region of the adjacent vertex of the major axis of the ellipse engages in this guide groove (29).

## Revendications

1. Conteneur de collecte et de déchets avec une partie inférieure de conteneur (1) et un couvercle (2) de conteneur comprenant:
- un orifice d'insertion (9),
- une sorte de cadre de support (13) qui dépasse du couvercle du conteneur (2) à la manière d'une tubulure et est destiné à et adapté pour un dispositif bordant l'orifice d'insertion, et
- un élément de fermeture (14) qui est prévu dans le dispositif bordant l'orifice d'insertion (9) du cadre de support au moins pour recouvrir l'orifice d'insertion en fermant l'orifice formé par le cadre de support, une position supérieure du dispositif étant prévue pour une fermeture provisoire de l'orifice d'insertion (9) et une position inférieure du dispositif obtenue pour l'élément de fermeture dans le cadre de support en exerçant une force à partir de la position supérieure étant prévue pour son recouvrement définitif,
- le cadre de support (13) et l'élément de fermeture (14) formant une unité de recouvrement et de fermeture (12) pour l'orifice d'insertion (9), les deux étant réalisés sous forme de pièces séparées et l'élément de fermeture (14) étant introduit dans le cadre de support (13) et maintenu à l'intérieur de manière à pouvoir être déplacé de la position supérieure à la position inférieure du dispositif,
- l'unité de recouvrement et de fermeture (12) étant disposée de manière à pouvoir tourner autour d'un axe de rotation sur le couvercle (2) du conteneur entre une position recouvrant l'orifice d'insertion (9) et une position dégageant au moins en grande partie l'orifice d'insertion (9), l'élément de fermeture (14) se trouvant dans sa position supérieure dans le cadre de support (13) pour effectuer ces mouvements,
- une sorte de cadre de fermeture (31) dépassant vers le bas à la manière d'une tubulure et épousant le contour de l'orifice d'insertion (9) étant réalisé sur la face inférieure du couvercle de conteneur (2) et
- l'élément de fermeture (14) entrant en prise dans le cadre de fermeture (31) par un tronçon terminal inférieur adapté au contour de l'orifice d'insertion (9) après avoir traversé l'orifice d'insertion (9) dans sa position inférieure pouvant être prise par le dispositif à partir de la position recouvrant l'orifice d'insertion (9) de l'unité de recouvrement et de fermeture (12), des configurations d'arrêt (32, 35) prévues au moins pour assurer un arrêt supplémentaire de l'élément de fermeture agissant sur le cadre de fermeture (31) et sur l'élément de fermeture (14),
**caractérisé en ce que**
- l'orifice d'insertion (9), le cadre de support (13) et l'élément de fermeture (14) présentent un contour elliptique qui leur est adapté et l'orifice d'insertion est disposé au bord du couvercle de conteneur (2) réalisé de forme elliptique de telle manière que leurs axes d'ellipse principaux coïncident et
- **en ce que** le conteneur a, en raison de sa section transversale elliptique, une forme plate qui permet de le manipuler avec une seule main à l'aide du pouce tandis que la main entoure le conteneur, en ce qui concerne la rotation de l'unité de recouvrement et de fermeture entre la position de recouvrement de l'orifice d'insertion et la position dégageant au moins en grande partie l'orifice d'insertion.

2. Conteneur selon la revendication 1, **caractérisé en ce que** l'élément de fermeture (14) est, par rapport à la face supérieure du couvercle de conteneur (2), réalisé en forme de capuchon avec une paroi latérale (23) perpendiculaire vers le bas sur la face extérieure de laquelle une configuration à cran (24, 25) est respectivement prévue dans les zones supérieure et inférieure du bord sur tout le pourtour horizontal.

3. Conteneur selon la revendication 2, **caractérisé en ce que**
- le cadre de support (13) est formé par une paroi (19) surélevée verticalement par rapport au couvercle de conteneur (2), ressemblant à un cercle de fût, sur le bord supérieur de laquelle est réalisé un élargissement (22) dirigé vers l'intérieur comme une bride est réalisé, et
- **en ce qu'**une configuration à cran supérieure et inférieure (20, 21) est également réalisée sur la face intérieure du cadre de support (13) de façon complémentaire aux deux configurations à cran (24, 25) de l'élément de fermeture (14).

4. Conteneur selon la revendication 3, **caractérisé en ce que** qu'une troisième configuration à cran (32) est réalisée en complément de la configuration à cran inférieure (25) de l'élément de fermeture (14) sur la face intérieure du cadre de fermeture (31), à une distance déterminée par les deux configurations à cran (20, 21) du cadre de support (13) par rapport à la configuration à cran inférieure (21) de ce dernier.

5. Conteneur selon la revendication 4, **caractérisé en ce qu'**une quantité de surfaces de support (33, 34, 35) pour le bord inférieur de l'élément de fermeture (14) sont réalisées sous la forme de saillies de matériau dépassant légèrement de la paroi intérieure du cadre de fermeture (31) à distance en dessous de la configuration à cran (32).

6. Conteneur selon la revendication 5, **caractérisé en ce qu'**au moins une configuration (11) de séparation de canule (11) est prévue en dessous des surfaces de support (33, 34, 35) dans une saillie de matériau (10) plus grande que ces dernières.

7. Conteneur selon l'une des revendications précédentes, **caractérisé en ce que** l'unité de recouvrement et de fermeture est guidée dans deux glissières réalisées l'une en face de l'autre sur le couvercle du conteneur.

8. Conteneur selon l'une des revendications précédentes, **caractérisé en ce que** l'unité de recouvrement et de fermeture (12) est maintenue dans sa position recouvrant l'orifice d'insertion (9) par une configuration de serrage pouvant être surmontée en faisant usage de la force.

9. Conteneur selon l'une des revendications 1 à 8, **caractérisé en ce qu'**une languette (15) de la face inférieure de laquelle fait saillie verticalement vers le bas un pivot rotatif (16) qui est enfoncé dans un évidement correspondant du couvercle de conteneur (2), dépasse du bord inférieur du cadre de support (13).

10. Conteneur selon la revendication 9, **caractérisé en ce que**
- sur le pivot rotatif (16) un bourrelet d'étanchéité (17) qui coopère avec la paroi de l'évidemment, est réalisé sur le tronçon avec lequel il traverse l'évidemment dans le couvercle de conteneur (2) et
- **en ce que** l'extrémité libre (18) du pivot rotatif (16) est élargie pour s'accrocher derrière la face inférieure du couvercle de conteneur (2) puis prend une forme conique, le tronçon terminal libre comportant une fente réalisée au milieu dans la direction de l'axe.

11. Conteneur selon l'une des revendications précédentes, **caractérisé en ce que** sur le couvercle de conteneur (2) une glissière (26) réalisée au moins en forme d'arc de cercle est réalisée pour l'unité de recouvrement et de fermeture (12), l'axe de rotation (16) de l'élément de recouvrement et de fermeture (12) passant verticalement à travers le point central de l'arc de cercle.

12. Conteneur selon l'une des revendications 1 à 11, **caractérisé en ce que**
- l'axe de rotation (16) de l'unité dé recouvrement et de fermeture (12) est à proximité immédiate de l'une des deux crêtes secondaires de ellipse du couvercle de conteneur (2).

13. Conteneur selon la revendication 12, **caractérisé en ce que**
- la glissière (26) de l'unité de recouvrement et de fermeture (12) est formée par une sorte de nervure de matériau (27) qui en dépasse approximativement sur la moitié du périmètre du bord du couvercle de conteneur (2) situé en face de l'évidement pour le pivot rotatif (16), avec un élargissement (28) ressemblant à une nervure qui dépasse de son bord supérieur en direction du milieu de l'arc de cercle et forme avec la face supérieure du couvercle de conteneur (2) une rainure de guidage (29) et
- **en ce qu'**une saillie de matériau (30) qui dépasse du bord du cadre de support (13) dans la zone de la crête principale de l'ellipse voisine comme un nez s'engage dans cette rainure de guidage (29).
